# EUROPÄISCHE PATENTSCHRIFT

(11) **EP 1 342 070 B1**
(45) Veröffentlichungstag und Bekanntmachung des Hinweises auf die Patenterteilung: **20.01.2010**
(21) Anmeldenummer: 01985815.8
(22) Anmeldetag: 17.11.2001
(51) Int. Cl.: G01N 21/35, G01M 3/38, G01M 3/04, G01M 3/20, G01N 33/00

(54) **VERFAHREN ZUR FESTSTELLUNG EINES GASES MIT HILFE EINES INFRAROT-GAS-ANALYSATORS SOWIE FÜR DIE DURCHFÜHRUNG DIESES VERFAHRENS GEEIGNETER GASANALYSATOR**
METHOD FOR DETECTING A GAS USING AN INFRARED GAS ANALYZER AND GAS ANALYZER SUITABLE FOR CARRYING OUT SAID METHOD
PROCEDE POUR DETERMINER UN GAZ A L'AIDE D'UN ANALYSEUR DE GAZ A INFRAROUGE ET ANALYSEUR DE GAZ PERMETTANT DE METTRE EN OEUVRE CE PROCEDE

(30) Priorität: 13.12.2000 DE 10062126
(43) Veröffentlichungstag der Anmeldung: 10.09.2003
(73) Patentinhaber: Inficon GmbH, 50968 Köln (DE)
(72) Erfinder: KILIAN, Ralf, 50999 Köln (DE); ROLFF, Randolf, 50169 Kerpen-Horrem (DE); KÜSTER, Gerd, 51109 Köln (DE); HIRCHE, Ralf, 51101 Köln (DE)
(74) Vertreter: Leineweber, Jürgen
(86) Internationale Anmeldenummer: PCT/EP2001/013298
(87) Internationale Veröffentlichungsnummer: WO 2002/048686

(56) Entgegenhaltungen:
- EP-A- 0 405 841
- WO-A2-99/14576
- DE-A- 19 911 260
- DE-C1- 4 012 454
- US-A- 4 393 304

## Beschreibung

Die Erfindung bezieht sich auf ein Verfahren zur Feststellung eines an einem Messort möglicherweise vorhandenen Testgases mit den Merkmalen des Oberbegriffs der Patentansprüche 1 sowie auf einen für die Durchführung dieses verfahrens geeigneten Infrarot-Gasanalysator.

Verfahren und Vorrichtungen dieser Art sind aus der DE-A-199 11 260 bekannt. Sie sind insbesondere für die Anwendung bei der Schnüffellecksuche geeignet. Bei der Schnüffellecksuche wird ein Nutzgas enthaltender Prüfling mit Hilfe einer Meßgase aufnehmenden Schnüffelspitze abgetastet. Ist ein Leck vorhanden, dringt Nutzgas nach außen. Dieses wird über die Schnüffelspitze dem Gasdetektor zugeführt. Ist das Nutzgas nicht infrarotaktiv, wird dem Nutzgas im Prüfling ein infrarotaktives Testgas hinzugefügt. In diesem Fall ist das durch ein eventuell vorhandenes Leck dringende Meßgas ein Gemisch aus Nutzgas und Testgas. Ist das Nutzgas bereits selbst infrarotaktiv (z.B. ein Halogengas), kann es selbst das Testgas (bzw. Meßgas) sein.

Bei der Schnüffellecksuche besteht das Problem, dass von der Schnüffelspitze nicht nur aus einem eventuell vorhandenen Leck (Messort) austretende Testgase sondern auch Gase aus der Umgebung des Messortes angesaugt werden. Enthalten diese bereits geringe Konzentrationen des Testgases, das zum Beispiel aus früher festgestellten Lecks oder aus Füllstationen einer Produktionslinie stammen kann, so werden diese ebenfalls vom Gasdetektor registriert. Dieses kann bei hohen Testgasuntergründen zu Fehlmessungen führen, d. h., dass dichte Prüflinge als fehlerhaft "erkannt" werden.

Um Nachteile dieser Art zu vermeiden, wird in der DE-A-199 11 260 vorgeschlagen, dass die Testgas-Konzentration des am Messort aufgenommenen Gases mit der Testgas-Konzentration des Referenzgases (in der Umgebung des Messortes aufgenommenes Gas) mit Hilfe von zwei Küvetten, einer Messküvette und einer Referenzküvette, verglichen wird, damit störende Einflüsse berücksichtigt werden können. Die Verwendung von zwei separaten Küvetten mit einer oder zwei exakt modulierten IR-Lichtquellen ist nicht nur technisch aufwendig, sie hat auch einige Nachteile. Einer dieser Nachteile besteht darin, dass die Küvetten ihre Eigenschaften nicht gleichmäßig verändern. Sie können ungleichmäßig verschmutzen; bei der Verwendung von zwei IR-Lichtquellen können diese unterschiedlich altern. In der zum Stand der Technik genannten Schrift wird zwar vorgeschlagen, dass es auch möglich ist, nur eine IR-Lichtquelle zu verwenden. Dieses macht aber eine Strahlteilung nötig. Diese Strahlteilung und auch die in der genannten Schrift offenbarte Strahlzusammenführung (zum Zwecke der Verwendung nur eines IR-Detektors) haben relativ hohe Verluste (ca. 50 %) zur Folge, wodurch insbesondere die Empfindlichkeit des Gasanalysators beeinträchtigt ist.

Zum Stand der Technik gehört noch der Inhalt der EP-405 841 A2, der DE-40 12 454-C1 und der WO99/14574.

Die US-43 93 304 offenbart einen Gasanalysator, der nach dem Prinzip der Fluid-Modulation arbeitet. Er besitzt eine oder zwei Zellen. Für den Fall, dass nur eine Zelle vorhanden ist, wird ein Messgas und ein Vergleichsgas durch die Zelle geleitet. Beim Vergleichsgas handelt es sich um ein Null-Gas, d.h., es darf nicht mit dem zur analysierenden Gas verseucht sein.

Die EP-405 841 A2 beschreibt einen Gasanalysator, bei dem die Strahlung einer Strahlungsquelle die Strömung eines zu analysierenden Gases durchsetzt. Es wird abwechselnd das zu analysierende Gas und ein Referenzgas zugeführt. Die Konzentration des Referenzgases ist konstant und ist wesentlich höher als die Hälfte des vollen Messbereichs.

Die DE-40 12 454-C1 (D3) sowie WO99/14574 (D$) offenbaren Verfahren und Vorrichtungen zur Feststellung von Isotopen-Verhältnissen. Den jeweiligen Einrichtungen werden Messgase und Referenzgase zugeführt.

Der vorliegenden Erfindung liegt die Aufgabe zugrunde, Verfahren und Vorrichtungen der hier betroffenen Art zu vereinfachen und insbesondere in Bezug auf ihre Empfindlichkeit zu verbessern.

Erfindungsgemäß wird diese Aufgabe durch die kennzeichnenden Merkmale der Patentansprüche gelöst.

Bei den erfindungsgemäßen Verfahren und Vorrichtungen wird nur eine Küvette benötigt. Verschmutzungen und Änderungen des gesamten Strahlenganges (Lampe-Küvette-Detektor) wirken sich gleichermaßen während des Messgaszyklus und während des Referenzgaszyklus aus. Die einzige IR-Lichtquelle muss nicht unbedingt moduliert werden, die Modulation wird durch den Gasaustausch erzielt. Vor allem entfällt die beim Stand der Technik notwendige, besonders exakte Modulation d.h., es können langsamere und hellere Lichtquellen verwendet werden, was sich besonders vorteilhaft auf die Empfindlichkeit des Analysators auswirkt. Auch eine Strahlteilung und eine Strahlenzusammenführung entfallen. Die einzige Küvette ist ständig mit dem möglicherweise Testgas enthaltenden Messgas und zeitweise zusätzlich mit Referenzgas gefüllt. Wenn die Konzentration des Testgases im Messgas, also am Messort, höher ist als die Konzentration des Testgases im Referenzgas, detektiert der IR-Detektor ein Wechselsignal, das ein Maß für die Konzentrationsdifferenz ist. Dabei spielt es keine Rolle, ob tatsächlich ein Testgasuntergrund vorhanden ist oder nicht. Daraus ergibt sich schließlich der Vorteil, dass beim Gegenstand der Erfindung die Nulllinie (Messgas = Referenzgas) wesentlich stabiler als beim Stand der Technik ist, weil der Gasaustausch für den Fall, dass Messgas und Referenzgas identisch sind, keine Modulationskomponente erzeugt. Demgegenüber müssen beim Stand der Technik zwei relativ große modulierte Signale miteinander verglichen werden, was im allgemeinen mit nicht unerheblichen Störkomponenten verbunden ist.

Im Rahmen der Erfindung ist es vorteilhaft, Maßnahmen zur Überwachung der Funktionsfähigkeit des IR-Gasanalysators nach der Erfindung vorzusehen, um durch Verschmutzungen oder Defekte verursachte Fehlmessungen zu vermeiden. Durch Verschmutzungen kann nicht nur die Gaszuführung gestört sein; auch die Empfindlichkeit des Gasanalysators selbst nimmt mit zunehmender Verschmutzung ab.

Zur Vermeidung eines gestörten Gaszuflusses wird vorgeschlagen, den Druck in den Gaszuführungsleitungen als Meßgröße zur Flußüberwachung zu verwenden. Die Überwachung der Funktion des Gasanalysators selbst erfolgt erfindungsgemäß in der Weise, dass die IR-Lichtquelle mit einer Referenzfrequenz moduliert wird, dessen Signal am IR-Detektor laufend überwacht wird (vorzugsweise mit einer eigenen Lock-In-Auswertung).

Besonders zweckmäßig ist es schließlich, als IR-Lichtquelle eine Gaslampe zu verwenden, die Testgas - zumindest anteilig - enthält. Gaslampen haben gegenüber Lampen mit einer Glühemission die generellen Vorteile, dass sie heller (bessere Ausnutzung der Lichtleistung) und schneller schaltbar - modulierbar - sind. Da ein IR-Detektor ein begrenztes Signal-/Rausch-Verhältnis hat, wächst die Auflösung des Analysators mit der Helligkeit der Lichtquelle.

Als Gaslampen können Blitzlampen, Gasentladungslampen, Glimmlampen oder dgl. verwendet werden. Da sie mit dem Gas betrieben werden, das detektiert werden soll, können Filter entfallen. Weiterhin kann ein großer Bereich des Absorptionsspektrums genutzt werden, weil das erzeugte Spektrum und das Spektrum des Meßgases weitgehend übereinstimmen. Der Anteil der genutzten Lichtleistung ist hoch, wodurch sich die maßgeblich verbesserte Gasselektivität des IR-Detektors ergibt. Die Umstellung auf eine andere Gasart kann in einfacher Weise dadurch geschehen, dass eine Gaslampe mit der anderen Gasart eingesetzt wird.

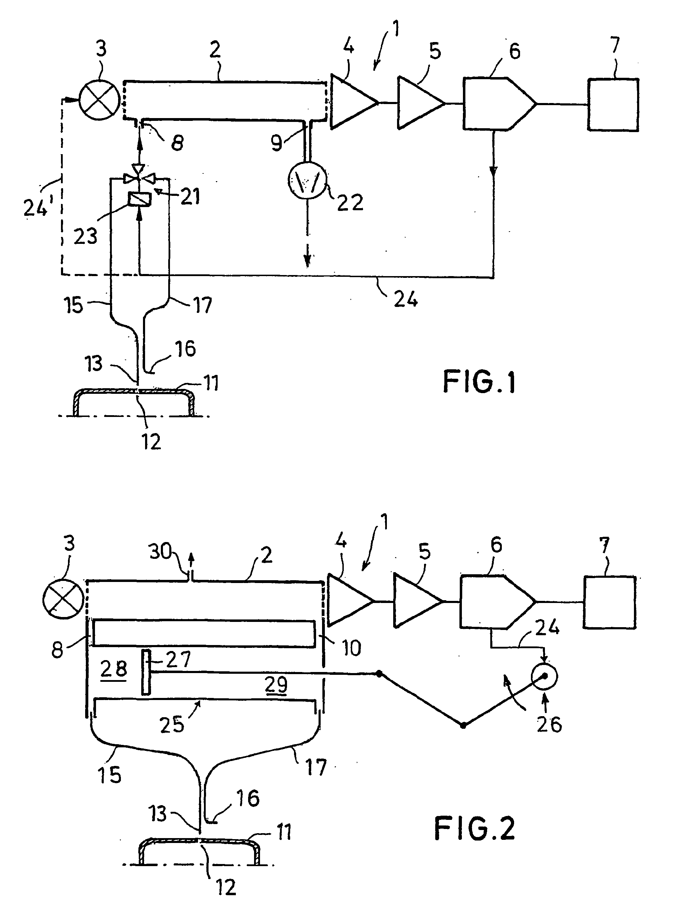

Weitere Vorteile und Einzelheiten der Erfindung sollen an Hand von in den Figuren 1 bis 6 dargestellten Beispielen und Ausführungen erläutert werden.

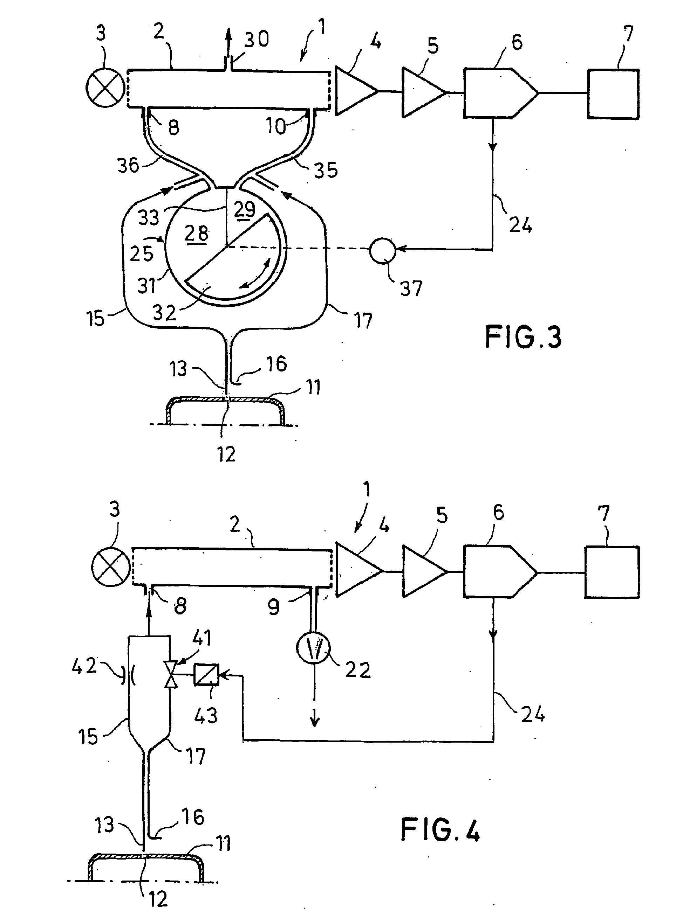

Die Figuren 1 bis 4 zeigen Infrarot-Gasanalysatoren mit unterschiedlichen Einrichtungen für die Zuführung des Messgases und des Referenzgases zur einzigen Küvette. Dieses geschieht
- im nicht erfindungsgemäßen Beispiel nach Figur 1 mit Hilfe eines Ventilsystems,
- im nicht erfindungsgemäßen Beispiel nach Figur 2 über ein Zwischenvolumen mit einem sich linear bewegenden Kolben,
- im nicht erfindungsgemäßen Beispiel nach Figur 3 über ein Zwischenvolumen mit einem Dreh-Schwingkolben und
- in der Ausführung nach Figur 4 über eine Drossel (Messgas) und ein Ventil (Referenzgas).

Figur 5 zeigt ein Beispiel mit einer Schnüffelflussüberwachung.

In Figur 6 ist ein Beispiel dargestellt, das mit Mitteln zur Überprüfung seiner Funktionsfähigkeit ausgerüstet ist.

In allen Figuren sind der Infrarot-Gasanalysator allgemein mit 1, seine Küvette mit 2, die auf einer Stirnseite angeordnete IR-Lichtquelle mit 3, der auf der gegenüberliegenden Stirnseite angeordnete Infrarot-Detektor mit 4, ein sich anschließender elektronischer Baustein (Verstärker/Filter) mit 5, ein sich daran anschließender weiterer, der Signalverarbeitung dienender elektronischer Baustein (Lock-in-Verstärker) mit 6 und eine Anzeige mit 7 bezeichnet. Die Lock-In-Verarbeitung wird meistens softwaremäßig in einem Microcontroler durchgeführt; nur der besseren Erläuterung wegen ist ein separater Block 6 dargestellt. Die Küvette 2 ist mit jeweils stirnseitig angeordneten Anschlüssen 8 und 9 ausgerüstet, über die nach den weiter unten beschriebenen Verfahren Messgas und Referenzgas zugeführt bzw. abgeführt wird.

Die Stirnseiten der Küvette 2 sind jeweils gestrichelt dargestellt. Dadurch soll zum Ausdruck gebracht werden, dass die an sich gasdichten Wandungen der Küvette im Bereich ihrer Stirnseiten für Infrarot-Licht durchlässig sind. Als Infrarot-Lichtquellen und -Detektoren können Einrichtungen verwendet werden, wie sie in der DE-A-199 11 260 beschrieben sind.

Bei allen Beispielen ist als Applikation des Gegenstandes der Erfindung die Schnüffellecksuche gewählt worden. Mit 11 ist ein auf Lecks zu untersuchender Prüfling bezeichnet, und zwar mit einem Leck 12. Der Ort des Lecks ist in diesem Fall der Messort. Die Schnüffelspitze 13 dient der Aufnahme des Messgases, das wegen des Vorhandenseins des Lecks 12 Testgas enthält. Über die sich an die Schnüffelspitze anschließende Leitung 15 strömt das aufgenommene Messgas zur Küvette 2. Der Aufnahme von Gas aus der Umgebung der Schnüffelspitze (Referenzgas) dient die Öffnung 16 der Schlauchleitung 17. Dieses Gas kann einen Testgasuntergrund enthalten, der bei der Feststellung der Konzentration des aus dem Leck 12 ausströmenden Gases berücksichtigt werden soll.

Bei dem Beispiel nach Figur 1 dient ein Steuerventil 21 der wechselweisen Zuführung des Messgases und des Referenzgases. Es ist so ausgebildet, dass entweder die Leitung 15 oder die Leitung 17 mit dem Einlass-Anschluss 8 der Küvette 2 in Verbindung steht. Das jeweilige Gas durchströmt die Küvette in axialer Richtung und verläßt sie durch den Auslass-Anschluss 9, der mit einer Förder- bzw. Vakuumpumpe 22 in Verbindung steht. Diese bestimmt je nach Saugvermögen die Strömungsgeschwindigkeit der zu untersuchenden Gase in der Küvette 2.

Der Gaswechsel erfolgt vorzugsweise periodisch. Dazu steht die Steuereinheit 23 des Steuerventils 21 über die Leitung 24 mit dem Lock-in-Verstärker 6 in Verbindung. Die Verwendung der an sich bekannten Lock-In-Technik hat den Vorteil, dass das Nutzsignal frequenz- und phasenselektiv gefiltert wird. Störsignale werden dadurch sehr effektiv unterdrückt. Für den Fall, dass auch die IR-Lichtquelle 3 synchron zum Gaswechsel moduliert werden soll, steht auch diese mit dem Lock-in-Verstärker 6 in Verbindung. Diese Variante ist durch die gestrichelte Linie 24 angedeutet. Die Lock-In-Verarbeitung incl. der Steuerung kann auch von einem Microcontroler-System mit geeigneter Software übernommen werden.

Bei dem Beispiel nach Figur 2 befindet sich zwischen der Küvette 2 und den Leitungen 15, 17 ein vorzugsweise - wie dargestellt - zylindrisch ausgebildetes Zwischenvolumen 25, in dem sich ein mit einem Kurbelantrieb 26 in Verbindung stehender Kolben 27 befindet. Die Leitungen 15 und 17 münden jeweils im Bereich der gegenüberliegenden Stirnseiten des Zwischenvolumens 25. In diesen Bereichen ist das Zwischenvolumen auch mit den Einlass-Anschlüssen 8 und 10 an der Küvette 2 verbunden. Der Kolben 27 bildet im Zwischenvolumen zwei getrennte Kammern 28, 29. Kammer 28 dient der Aufnahme und Abgabe von Messgas, Kammer 29 der Aufnahme und Abgabe von Referenzgas. Die Hin- und Herbewegung des Kolbens 25 bewirkt eine wechselweise Zuführung von Messgas und Referenzgas in die Küvette 2. Die zugeführten Gase verlassen die Küvette über den zweckmäßig etwa in der Mitte der Küvette 2 angeordneten Auslass-Anschluss 30, der mit der Pumpe 22 (Fig. 1) verbunden ist. Im Vergleich zur Ausführung nach Figur 1 findet ein schnellerer und vollständigerer Gasaustausch in der Küvette 2 statt.

Auch bei dem Beispiel nach Figur 3 ist ein Zwischenvolumen 25 mit den Kammern 28, 29 vorhanden. Diese werden gebildet von einem im Querschnitt kreisförmigen Gehäuse 31, einem im Querschnitt halbkreisförmigen Dreh-Schwing-Kolben 32 und einer radialen Trennwand 33. Die Kammern 28, 29 stehen über die Leitungen 35, 36 mit den Anschlüssen 8, 9 der Küvette 2 in Verbindung. In die Leitungen 35, 36 münden die Leitungen 15, 17 derart, dass eine Schwingbewegung des Kolbens 32, erzeugt vom Antrieb 37, die Küvette 2 abwechselnd mit Messgas und mit Referenzgas füllt. Diese Gase verlassen die Küvette 2 über den mittleren Anschluss 30.

Der Takt, mit dem der periodische Gaswechsel in der Küvette 2 bei den Ausführungen nach den Figuren 1, 2 und 3 stattfindet, bestimmt der Lock-in-Verstärker 6. Dieser steht jeweils über die Leitung 24 mit dem Ventil 21, dem Kurbelantrieb 26 oder dem Antrieb 37 des Schwingkolbens 32 in Verbindung. Als zweckmäßig haben sich Taktzeiten von 1 sec bis 1/6 sec pro Periode erwiesen.

Bei den Beispielen nach den Figuren 1 bis 3 ist die Küvette 2 in einer ersten Zeitperiode mit dem Messgas und in einer zweiten Zeitperiode mit dem Referenzgas gefüllt. Im Gegensatz dazu wird bei der Ausführung nach Figur 4 das Messgas der Küvette 2 über die Leitung 15 kontinuierlich zugeführt. Referenzgas wird nur periodisch zugeführt, und zwar über das in der Leitung 17 angeordnete Ventil 41 mit seiner Steuereinheit 43. Die Öffnungs- und Schließzeiten dieses Ventils werden ebenfalls durch den vom Lock-in-Verstärker 6 vorgegebenen Takt bestimmt. Während des Messbetriebes ist die Küvette 2 abwechselnd von Referenzgas und Messgas bzw. nur Messgas durchströmt. Enthält das Messgas aus einem Leck ausgetretenes Testgas, liefert der Detektor 4 das gewünschte Wechselsignal.

Da die Leckratenempfindlichkeit vom Messgas- bzw. Testgasfluss abhängt (hohe Empfindlichkeit bei kleinem Messgasfluss), ist es zweckmäßig, die Leitung 15 mit einer Drossel 42 auszurüsten, die so bemessen ist, dass der Messgasfluss die Küvette in einer halben Periode gerade mit Meßgas füllt. Demgegenüber darf der Referenzgasfluss hoch sein, da praktisch genügend Referenzgas zur Verfügung steht.

Weiterhin besteht bei allen Ausführungen die Möglichkeit, den Referenzgasmesszyklus kürzer zu wählen als den Messgasmesszyklus. Dadurch verkürzt sich die Totzeit, schnellere und/oder empfindlichere Messungen sind möglich.

Wie bereits erwähnt, muß die IR-Lichtquelle nicht mit dem Takt des Lock-in-Verstärkers moduliert sein, da bereits der Gaswechsel die gewünschte Modulation bewirkt. Es besteht jedoch die Möglichkeit, die IR-Lichtquelle 3 zusätzlich synchron zum Gaswechsel zu modulieren, um steilere Flanken der Messsignale zu erreichen. Ohne Modulation sind die Messsignalflanken durch die Schnelligkeit des Gaswechsels bestimmt.

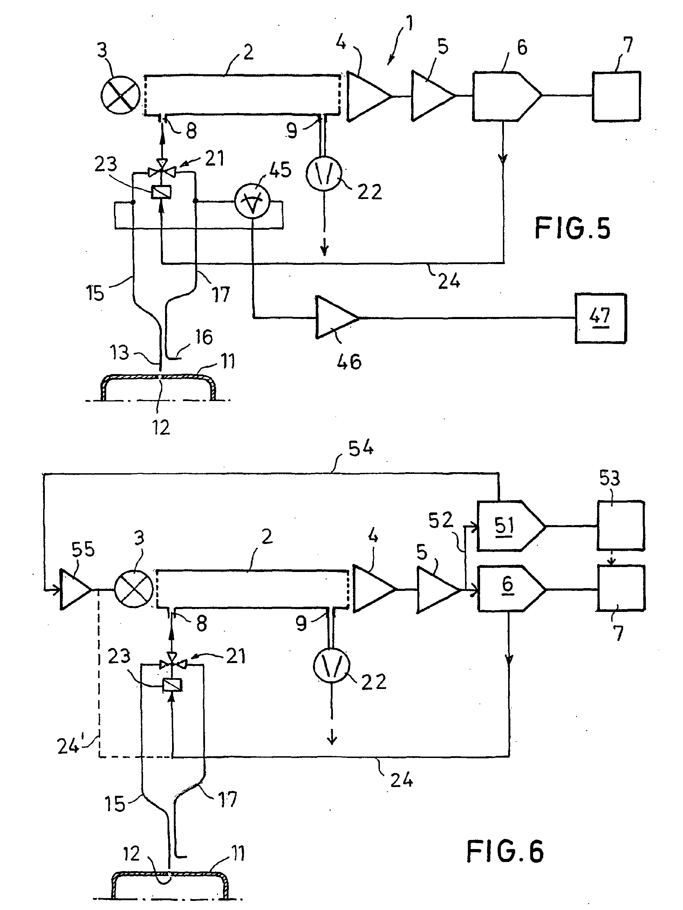

Figur 5 zeigt anhand des Beispiels nach Figur 1, wie der Schnüffelfluss mit hoher Empfindlichkeit während des Betriebs überprüft/überwacht werden kann. Dieses geschieht mit Hilfe eines Differenzdrucksensors 45, der in der Höhe des Ventils 21 mit den Leitungen 15,17 in Verbindung steht. Das gemessene Differenzdrucksignal wird über einen Verstärker 46 einer Auswertelogik 47 zugeführt. Wenn man dieses Differenzdrucksignal phasenselektiv zur Umschaltfrequenz auswertet, erhält man die Information darüber, ob der Fluss der einen oder der anderen Schnüffelleitung oder beider Schnüffelleitungen sich geändert hat. Auch eine Verstopfung der Leitung der Pumpe bzw. ein Defekt der Pumpe wird erfasst.

Eine andere Möglichkeit der Differenzdrucküberwachung kann mit Hilfe von Flusssensoren realisiert werden. Sie ist jedoch aufwendiger als die vorbeschriebene Methode.

Figur 6 zeigt ebenfalls anhand des Beispiels nach Figur 1, wie die Funktion des Gasanalysators 1 mit hoher Empfindlichkeit überprüft/überwacht werden kann. Sie ist anwendbar bei einer Lösung, bei der das Signal am Sensor mit einer Grundfrequenz fg (geliefert vom Lock-in-Verstärker 6) moduliert ist und bei der die frequenzselektive Empfangseinheit die Leckrate ermittelt.

Bei der Lösung nach Figur 6 ist ein zweiter Lock-in-Verstärker 51 vorgesehen, der einen Referenztakt mit einer Frequenz fr liefert. Die Eingänge der Lock-in-Verstärker 6 und 51 sind über die Leitung 52 miteinander verbunden. An den Auslass des Lock-in-Verstärkers 51 schließt eine Auswerteeinheit 53 an, die ihre Informationen an die Anzeige 7 weitergibt. Über die Leitung 54 mit dem Verstärker 55 steht der Lock-in-Verstärker 51 mit der IR-Lichtquelle 3 in Verbindung.

Bei dem in Figur 6 dargestellten Beispiel erfolgt die Überwachung des Gasanalysators 1 in der Weise, dass die Infrarotlampe 3 mit einem (zusätzlichen) Signal anderer Frequenz moduliert wird. Um Interferenzen mit dem Nutzsignal fg zu vermeiden, bietet sich eine Frequenz von z.B. fr=2,5 fg an. Die Modulationsamplitude muss so groß gewählt werden, dass man dieses Signal in der Empfangseinheit frequenzselektiv gut auswerten kann. Man erhält dann ein Messsignal mit der Frequenz fg und ein Referenzsignal mit der Frequenz fr, die voneinander unabhängig auswertbar sind. Sollte nun die Systemempfindlichkeit durch einen Fehler abnehmen, erkennt man dies an der abnehmenden Amplitude des Signalanteils bei fr (auch wenn kein Leck gemessen wird). Hieraus kann man Grenzen für eine Fehlermeldung festlegen.

Die Auswertung erfolgt im Block 53. Diese kann auch dazu dienen, bei abnehmender Empfindlichkeit den Kalibrierfaktor anzupassen und damit die Messgenauigkeit zu erhöhen.

In den Zeichnungen und Beschreibungen sind jeweils separate Blöcke dargestellt, die Bestandteile der verwendeten Schaltungen sind. Zeckmäßig ist die Verwendung integrierter Systeme. Beispielsweise können zur Lock-in-Verarbeitung, Steuerung und Verarbeitung der Mess- und Steuersignale ein Microcomputer- bzw. eine Microprozessorschaltung mit zugehöriger Software verwendet werden.

## Patentansprüche

1. Verfahren zur Durchführung der Schnüffellecksuche, bei der zur Feststellung eines an einem Messort möglicherweise vorhandenen Testgases ein Infrarot-Gasanalysator (1) verwendet wird; wobei der Infrarot-Gasanalysator (1) eine einzige die zu untersuchenden Gase aufnehmende Küvette (2), eine auf einer Stirnseite der Küvette angeordnete IR-Lichtquelle (3), einen auf der anderen Stirnseite der Küvette angeordneten IR-Detektor (4) sowie der steuerung und Signalverarbeitung dienende elektronische Bausteine, deren Signale der Festellung von am Messort aufgenommenem Testgas dienen umfasst; die Zuführung von Gasen zum Infrarot-Gasanalysator (1) über zwei Gasleitungen (15, 17) erfolgt; wobei über eine erste Gasleitung (15) die Zuführung von Messgas erfolgt, das am Messort aufgenommen wird und möglicherweise Testgas enthält; über die zweite Leitung (17) Referenzgas zugeführt wird, das in der Umgebung des Messortes aufgenommen wird und einen Testgasuntergrund enthalten kann, der bei der Feststellung des am Messort aufgenommenen Testgases berücksichtigt wird;
und wobei das Verfahren **dadurch gekennzeichnet ist,**
**dass** das am Messort aufgenommene Messgas und das in der Umgebung des Messortes aufgenommene Referenzgas in die Küvette (2) des Infrarot-Gasanalysators derart eingelassen werden, dass die Küvette (2) ständig von am Messort aufgenommenem Messgas und periodisch zusätzlich von Referenzgas durchströmt ist, und dass für den Fall, dass am Messort Testgas aufgenommen wird, dessen Konzentration im Messgas höher ist als die Testgaskonzentration im Referenzgas, aus den vom IR-Detektor gelieferten Wechselsignalen die erhöhte Testgaskonzentration festgestellt wird.

2. Verfahren nach Anspruch 1, **dadurch gekennzeichnet, dass** zur Signalauswertung die Lock-in-Technik angewendet wird, indem für die periodische Zuführung des Referenzgases ein Lock-in-Verstärker verwendet wird.

3. Verfahren nach einem der Ansprüche 1 oder 2, **dadurch gekennzeichnet, dass** der Meßzyklus länger gewählt wird als der Referenzzyklus.

4. Verfahren nach einem der Ansprüche 1 bis 3, **dadurch gekennzeichnet, dass** die Funktionsfähigkeit des Gasanalysators laufend überwacht wird.

5. Verfahren nach einem der Ansprüche 1 bis 4, **dadurch gekennzeichnet, dass** der Gaszufluß laufend überwacht wird.

6. Verfahren nach Anspruch 5, **dadurch gekennzeichnet, dass** der Druck in den Zuführungsleitungen (15, 17) gemessen wird und dass die Differenz dieser Drücke als Meßgröße dient.

7. Verfahren nach einem der vorhergehenden Ansprüche, **dadurch gekennzeichnet, dass** auch die IR-Lichtquelle (3) synchron zum Gaswechsel moduliert wird.

8. Vorrichtung zur Durchführung der Schnüffellecksuche nach einem Verfahren mit den Merkmalen des Anspruchs 1, **dadurch gekennzeichnet, dass** sich in der der Zuführung von Referenzgas dienenden Leitung (17) ein von einer mit einem vorgegebenen Takt beaufschlagten Skeuereinheit (43) angesteuertes Steuerventil (41) befindet, über das der ständig von am Messort aufgenommenem Messgas durchströmten Küvette (2) zusätzlich periodisch Referenzgas zugeführt wird und dass die Vorrichtung einen der Signalverabeitung dienenden elektronischen Baustein (6) umfasst, welcher aus den vom IR-Detektor gelieferten Wechselsignalen die erhöhte Testgaskonzentration feststellt.

9. Vorrichtung nach Anspruch 8, **dadurch gekennzeichnet, dass** die Küvette mit einem Auslass-Anschluss (9, 30) ausgerüstet ist, an den eine Förder-/Vakuumpumpe (22) angeschlossen ist.
Wechselsignalen die erhöble Testgashunzentration festtellt.

10. Vorrichtung nach Anspruch 8, **dadurch gekennzeichnet, dass** sich in der der Zuführung von Meßgas dienenden Leitung (15) eine Drossel (42) befindet.

11. Vorrichtung nach Anspruch 8, **dadurch gekennzeichnet, dass** der der Signalverarbeitung dienende elektronische Baustein einen Lock-In-Verstärker (6) umfasst, dessen den Takt vergebende Leitung (24), mit der Steuereinheit (43) verbunden ist.

12. Vorrichtung nach Anspruch 11, **dadurch gekennzeichnet, dass** ein weiterer Lock-in-Verstärker (51) vorgesehen ist, dessen den Takt für die Referenzfrequenz vorgebende Leitung (54) mit der IR-Lichtquelle verbunden ist.

13. Vorrichtung nach Anspruch 12, **dadurch gekennzeichnet, dass** die Referenzfrequenz fr größer, vorzugsweise um etwa den Faktor 2,5 größer ist als die Grundfrequenz fg.

14. Vorrichtung nach Anspruch 12 oder 13, **dadurch gekennzeichnet, dass** der weitere Lock-In-Verstärker (51) mit einer Auswerteeinheit (53) in Verbindung steht, die der Erkennung eines Systemfehlers dient.

15. Vorrichtung nach Anspruch 14, **dadurch gekennzeichnet, dass** die Auswerteeinheit (53) auch zur Anpassung eines Kalibrierfaktors verwendet wird.

16. Vorrichtung nach Anspruch 8 zur Durchführung eines Verfahrens mit den Merkmalen des Anspruchs 5 oder 6, **dadurch gekennzeichnet, dass** ein Differenzdrucksensor (45) an die Leitungen (15, 17) angeschlossen ist und daß der Ausgang des Drucksensors mit einer Auswertelogik (47) verbunden ist.

17. Vorrichtung nach Anspruch 16, **dadurch gekennzeichnet, dass** die Auswertelogik (47) einen eigenen Lock-in-Verstärker enthält.

18. Vorrichtung nach einem der Ansprüche 8 bis 17, **dadurch gekennzeichnet, dass** zur Lock-In-Verarbeitung, Steuerung und Verarbeitung der Mess- und Steuersignale ein Microcomputer- bzw. eine Microprozessorschaltung mit zugehöriger Software verwendet wird.

## Claims

1. Method for carrying out sniffer leak detection, in which an infrared gas analyser (1) is used to detect a gas to be tested, possibly existing at a measurement site; wherein the infrared gas analyser (1) comprises a single cuvette (2) receiving the gases to be examined, an IR light source (3) arranged on one end side of the cuvette, an IR detector (4) arranged on the other end side of the cuvette, and electronic modules used for the control and signal processing, the signals of which are used to detect a gas to be tested, acquired at the measurement site; gases are fed to the infrared gas analyser (1) through two gas lines (15, 17); wherein a gas to be measured, which is acquired at the measurement site and possibly contains the gas to be tested, is fed through a first gas line (15); reference gas, which is acquired in the vicinity of the measurement site and may contain a background of the gas to be tested, which is taken into account for detecting the gas to be tested, acquired at the measurement site, is fed through the second line (17); the method being **characterised in that** the gas to be measured, acquired at the measurement site, and the reference gas, acquired in the vicinity of the measurement site, are introduced into the cuvette (2) of the infrared gas analyser so that the gas to be measured, acquired at the measurement site, flows constantly through the cuvette (2) and reference gas additionally flows through it periodically, and **in that** if the gas to be tested is acquired at the measurement site with a concentration in the gas to be measured which is higher than the concentration of the gas to be tested in the reference gas, the increased concentration of the gas to be tested is detected from the alternating signals delivered by the IR detector.

2. Method according to Claim 1, **characterised in that** the lock-in technique is employed for the signal evaluation, by using a lock-in amplifier for the periodic supply of the reference gas.

3. Method according to one of Claims 1 or 2, **characterised in that** the measurement cycle is selected to be longer than the reference cycle.

4. Method according to one of Claims 1 to 3, **characterised in that** the functionality of the gas analyser is monitored continuously.

5. Method according to one of Claims 1 to 4, **characterised in that** the gas influx is monitored continuously.

6. Method according to Claim 5, **characterised in that** the pressure in the supply lines (15, 17) is measured, and **in that** the difference between these pressures is used as a measurement quantity.

7. Method according to one of the preceding claims, **characterised in that** the IR light source (3) is modulated synchronously with the gas alternation.

8. Device for carrying out sniffer leakage detection by a method having the features of Claim 1, **characterised in that** the line (17) used to supply reference gas contains a control valve (41), controlled by a control unit (43) which receives a specified clock signal, by means of which reference gas is periodically supplied additionally to the cuvette (2) through which the gas to be measured, acquired at the measurement site, flows constantly, and **in that** the device comprises an electronic module (6) used for the signal processing, which detects the increased concentration of the gas to be tested from the alternating signals delivered by the IR detector.

9. Device according to Claim 8, **characterised in that** the cuvette is equipped with an outlet connection (9, 30) to which a delivery/vacuum pump (22) is connected.

10. Device according to Claim 8, **characterised in that** the line (15) used to supply the gas to be measured contains a throttle (42).

11. Device according to Claim 8, **characterised in that** the electronic module used for the signal processing comprises a lock-in amplifier (6), whose line (24) giving the clock signal is connected to the control unit (43).

12. Device according to Claim 11, **characterised in that** a further lock-in amplifier (51) is provided, whose line (54) giving the clock signal for the reference frequency is connected to the IR light source.

13. Device according to Claim 12, **characterised in that** the reference frequency fr is higher, preferably higher by about a factor of 2.5, than the basic frequency fg.

14. Device according to Claim 12 or 13, **characterised in that** the further lock-in amplifier (51) is in communication with an evaluation unit (53) which is used to identify a system error.

15. Device according to Claim 14, **characterised in that** the evaluation unit (53) is also used to adapt a calibration factor.

16. Device according to Claim 8 for carrying out a method having the features of Claim 5 or 6, **characterised in that** a differential pressure sensor (45) is connected to the lines (15, 17), and **in that** the output of the pressure sensor is connected to evaluation logic (47).

17. Device according to Claim 16, **characterised in that** the evaluation logic (47) contains its own lock-in amplifier.

18. Device according to one of Claims 8 to 17, **characterised in that** a microcomputer or a microprocessor circuit with associated software is used for the lock-in processing, control and processing of the measurement and control signals.

## Revendications

1. Procédé destiné à la mise en oeuvre de la recherche de fuites par renifleur, dans lequel un analyseur de gaz à infrarouge (1) est utilisé pour détecter un gaz témoin éventuellement présent en un lieu de mesure ; l'analyseur de gaz à infrarouge (1) comportant une seule cuvette (2) recevant les gaz à examiner, une source de lumière infrarouge (3), disposée sur une face frontale de la cuvette, un détecteur à infrarouge (4), disposé sur une autre face frontale de la cuvette, ainsi que des modules électroniques, destinés à la commande et au traitement des signaux, dont les signaux sont destinés à signaler la détection de gaz témoin enregistré sur le lieu de mesure ; les gaz sont acheminés vers l'analyseur de gaz à infrarouge (1) par l'intermédiaire de deux conduites de gaz (15, 17) ; une première conduite de gaz (15) assurant l'acheminement du gaz de mesure, qui est enregistré sur le lieu de mesure et contient éventuellement le gaz témoin ; la deuxième conduite (17) assurant l'acheminement d'un gaz de référence, qui est enregistré dans l'environnement du lieu de mesure et qui peut contenir une base de gaz témoin, qui est prise en compte lors de la détection du gaz témoin enregistré sur le lieu de mesure ;
et le procédé étant **caractérisé**
**en ce que** le gaz de mesure, enregistré sur le lieu de mesure, et le gaz de référence, enregistré dans l'environnement du lieu de mesure, sont introduits dans la cuvette (2) de l'analyseur de gaz à infrarouge, de telle sorte que la cuvette (2) est traversée en permanence par le gaz de mesure enregistré sur le lieu de mesure et périodiquement en plus par le gaz de référence, et en ce que, pour le cas où sur le lieu de mesure est enregistré un gaz témoin dont la concentration dans le gaz de mesure est supérieure à la concentration de gaz témoin dans le gaz de référence, la concentration plus élevée en gaz témoin est constatée à partir des signaux alternatifs délivrés par le détecteur à infrarouge.

2. Procédé selon la revendication 1, **caractérisé en ce que** pour l'analyse des signaux, on applique la technique lock-in, par le fait qu'un amplificateur lock-in est utilisé pour l'acheminement périodique du gaz de référence.

3. Procédé selon la revendication 1 ou 2, **caractérisé en ce que** le cycle de mesure est choisi plus long que le cycle de référence.

4. Procédé selon l'une quelconque des revendications 1 à 3, **caractérisé en ce que** le bon fonctionnement de l'analyseur de gaz est contrôlé en continu.

5. Procédé selon l'une quelconque des revendications 1 à 4, **caractérisé en ce que** l'admission de gaz est contrôlée en continu.

6. Procédé selon la revendication 5, **caractérisé en ce que** la pression dans les conduites d'acheminement (15, 17) est mesurée et **en ce que** la différence entre ces pressions est utilisée comme grandeur de mesure.

7. Procédé selon l'une quelconque des revendications précédentes, **caractérisé en ce que** la source de lumière infrarouge (3) est également modulée de manière synchrone avec le changement de gaz.

8. Dispositif destiné à la mise en oeuvre de la recherche de fuites par renifleur selon un procédé avec les caractéristiques de la revendication 1, **caractérisé en ce que** dans la conduite (17) destinée à l'acheminement du gaz de référence est située une vanne pilote (41), qui est actionnée par une unité de commande (43), sollicitée selon une fréquence prédéfinie, et par l'intermédiaire de laquelle le gaz de référence est acheminé en plus périodiquement vers la cuvette (2) traversée en permanence par le gaz de mesure enregistré sur le lieu de mesure, et **en ce que** le dispositif comporte un module (6) électronique, qui est destiné au traitement des signaux et qui, à partir des signaux alternatifs délivrés par le détecteur à infrarouge, constate la concentration plus élevée en gaz témoin.

9. Dispositif selon la revendication 8, **caractérisé en ce que** la cuvette est munie d'un raccord de sortie (9, 30), auquel est raccordée une pompe de circulation/pompe à vide (22).

10. Dispositif selon la revendication 8, **caractérisé en ce qu'**un étranglement (42) est situé dans la conduite (15) destinée à l'acheminement du gaz de mesure.

11. Dispositif selon la revendication 8, **caractérisé en ce que** le module électronique, destiné au traitement des signaux, comporte un amplificateur lock-in (6), dont la ligne (24) donnant la fréquence est reliée à l'unité de commande (43).

12. Dispositif selon la revendication 11, **caractérisé en ce qu'**il est prévu un amplificateur lock-in (51) supplémentaire, dont la ligne (54) donnant la fréquence pour la fréquence de référence est reliée à la source de lumière infrarouge.

13. Dispositif selon la revendication 12, **caractérisé en ce que** la fréquence de référence (fr) est supérieure, de préférence 2,5 fois supérieure à la fréquence de base (fg).

14. Dispositif selon la revendication 12 ou 13, **caractérisé en ce que** l'amplificateur lock-in (51) supplémentaire est relié à une unité d'analyse (53), qui est destinée à détecter une erreur du système.

15. Dispositif selon la revendication 14, **caractérisé en ce que** l'unité d'analyse (53) est également utilisée pour l'ajustement d'un facteur de calibrage.

16. Dispositif selon la revendication 8 destinée à la mise en oeuvre d'un procédé avec les caractéristiques de la revendication 5 ou 6, **caractérisé en ce qu'**un capteur de différence de pression (45) est raccordé aux conduites (15, 17) et **en ce que** la sortie du capteur de pression est reliée à une logique d'analyse (47).

17. Dispositif selon la revendication 16, **caractérisé en ce que** la logique d'analyse (47) contient son propre amplificateur lock-in.

18. Dispositif selon l'une quelconque des revendications 8 à 17, **caractérisé en ce que** pour le traitement lock-in, la commande et le traitement des signaux de mesure et des signaux de commande, on utilise un microordinateur ou un circuit à microprocesseur avec le logiciel correspondant.
